# EUROPEAN PATENT SPECIFICATION

(11) **EP 2 203 115 B1**
(45) Date of publication and mention of the grant of the patent: **20.11.2019**
(21) Application number: 08800080.7
(22) Date of filing: 30.09.2008
(51) Int. Cl.: G16H 50/20

(54) **ELECTROCARDIOGRAM DERIVED APNOEA/HYPOPNEA INDEX**
APNOE-HYPOPNOE-INDEX AUS EINEM ELEKTROKARDIOGRAMM
INDICE D'APNÉE-HYPOPNÉE DÉRIVÉ PAR ÉLECTROCARDIOGRAMME

(30) Priority: 02.10.2007 AU 2007905367
(43) Date of publication of application: 07.07.2010
(73) Proprietor: Compumedics Medical Innovation Pty Ltd, Abbotsford, Victoria 3067 (AU)
(72) Inventor: NILSEN, Kris, Abbotsford, VIC 3067 (AU); ZILBERG, Eugene, Sandringham, VIC 3191 (AU)
(74) Representative: Handsome I.P. Ltd
(86) International application number: PCT/AU2008/001445
(87) International publication number: WO 2009/043087

(56) References cited:
- US-A- 5 913 308
- US-A1- 2003 055 348
- US-A1- 2006 041 201
- US-A1- 2007 032 733
- US-A1- 2007 032 733
- US-B1- 6 415 174

## Description

This invention relates to methods and apparatus for acquiring and analysing electrocardiograph data from a subject, particularly from a subject having sleep-disordered breathing, more particularly, sleep apnoea, even more particularly, obstructive sleep apnoea or central sleep apnoea.

Sleep apnoea (SA), including obstructive sleep apnoea (OSA) and/or central sleep apnoea (CSA), in which breathing is arrested, affects a significant proportion of the population. The effects of OSA are felt when the airways collapse and air cannot pass. The effects of CSA are felt when a subject does not inspire and expire air due to causes associated with the functioning of the central nervous system. Both OSA and CSA can result in inefficient sleep and further medical problems.

Both OSA and CSA are currently diagnosed in sleep laboratories by overnight polysomnographic (PSG) studies where a subject sleeps while having numerous electrodes attached to the body for measuring various physiological parameters. Such PSG studies are costly to undertake because they require a subject to sleep overnight in a clinic.

Some approaches have been made to develop apparatus and methods for measuring the occurrence and type of SA. Standard PSG rules for diagnosing SA require detection of the actual start times, durations and categories of individual respiratory events. This information is then used for evaluating the presence and severity of sleep apnoea by generating an apnoea-hypopnea index (AHI) [1]. Currently the most successful reported methods can detect if a subject has sleep apnoea (without reliable estimate of the AHI value) or if there is a sleep apnoea event during any given minute [2- 5]. For example, US 5,769,084 teaches a method of using chaotic processing of various sleep parameters, including measuring the electrocardiogram (ECG), for determining the presence of SA. However, the method does not extend to discriminating OSA from CSA. A method for measuring SA from sensors located on different planes on a subject is taught in US 6,415,174. This document does not teach a method that can discriminate between CSA and OSA. In WO 2005/067790, incorporated herein by reference, Burton indicated that it was possible to use an ECG trace to identify instances of SA and classify the SA as either OSA or CSA. However, the CSA signal is small relative to the background noise, i.e. there is a small signal-to-noise ratio.

There is a large prevalence of OSA and CSA among cardiac patients making the use of ECG data to determine the AHI an even more attractive option than the use of PSG data. What is needed is a more convenient and reliable method to measure sleep apnoea and its form using simpler apparatus than PSG studies. The methods should be able to detect and classify individual respiratory events. Preferably, such methods would be carried out in the home [6-8].

US2007032733 and US2006041201 each describe methods and apparatus for use in detecting sleep disordered breathing using an ECG.
Figure 1 shows an overview flow chart of the steps for calculating the hypopnea/apnoea index from ECG data complexes.
Figure 2 shows a flow chart of the detailed steps for calculating the hypopnea/apnoea index from ECG data complexes.
Figure 3 shows a graph of the output of a typical ECG lead I complex with each section of the graph labelled.
Figure 4 shows a plot of ECG signal against time of the QRS area, R- amplitude, and respiration flow rate of a subject displaying regular, steady breathing during sleep.
Figure 5 shows a plot of ECG signal against time of the QRS area, R- amplitude, and respiration flow rate of a subject displaying a respiratory event.
Figure 6 shows a graph of mV of the R-wave against QRS complex number in an ECG signal in a respiratory event during sleep.
Figure 7 shows a graph of mV of the R-R interval and flow rate of respiration against time against QRS complex number in an ECG signal over a series of respiratory events during sleep.
Figure 8 shows a graph of the signals against time for QRS area, R-amplitude in an ECG signal, and flow rate during periods of apnoea and normal breathing during sleep.
Figure 9 shows a graph of amplitude of the R-wave against QRS complex number in an ECG signal during a respiratory event.
Figure 10 shows a graph of area of QRS region against QRS complex number in an ECG signal during a respiratory event.
Figure 11 shows a graph of R-R interval against QRS complex number in an ECG signal over a respiratory event.
Figure 12 shows a graph of QRS area against QRS complex number through a respiratory event in an ECG signal.
Figure 13a shows the output signal showing the R-wave though a respiratory event in a first ECG lead.
Figure 13b shows the output signal showing the R-wave from a second ECG lead through the respiratory event shown in Fig 12a.
Figure 14 shows a graph of R-R interval against QRS number for an ECG signal through a respiratory event.
Figure 15 shows a plot of EDR signal against QRS complex number in an ECG signal through a respiratory event.
Figure 16 shows a graph of the amplitude of an R-wave against QRS complex number in an ECG signal through a respiratory event.
Figure 17a shows graphs of QRS area and R-wave amplitude in an ECG signal over time for an OSA event.
Figure 17b shows graphs of QRS area and R-wave amplitude in an ECG signal over time for a CSA event.

The present invention is defined by the appended claims.

### Detailed description

The present invention provides a previously unknown method for determining an apnoea/hypopnea index from ECG signal data acquired from a subject. The method of the invention advantageously exploits the variation in ECG signals acquired during sleep to calculate an apnoea/hypopnea index for the period of sleep.

Different ECG leads measure a difference in electro-potentials across different regions of the body. Breathing affects the impedance between electrodes and therefore the potentials and the differences among them. The movement of the chest and abdomen changes during respiratory events and therefore changes how the impedance between electrodes changes with breathing effort. This results in a phase change between ECG derived signals that can be observed.

The method uses the phase changes as markers for respiratory events. The method advantageously exploits the short period after a respiratory event and during an arousal where there is short period of increased heart rate, systolic blood pressure, and diastolic blood pressure accompanied by increased sympathetic activity. Apnoea events are usually terminated with an arousal enabling the markers of an arousal to be used as markers for apnoea termination. The burst of increased heart rate is easily detectable from the ECG. Sudden drops in the R-R interval as described below are used as markers for the end of respiratory events.

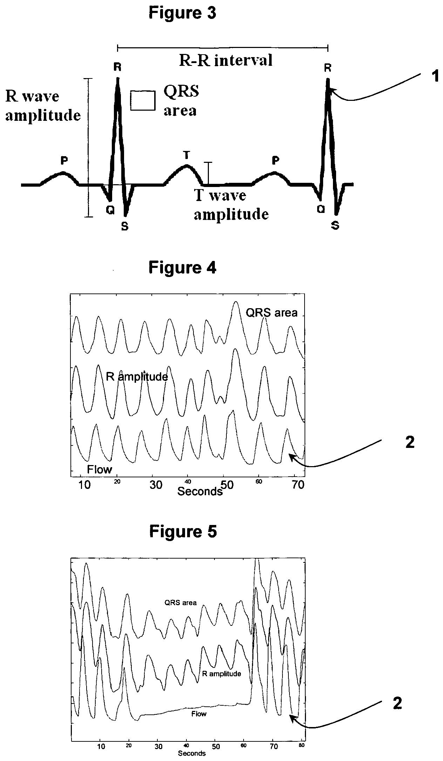

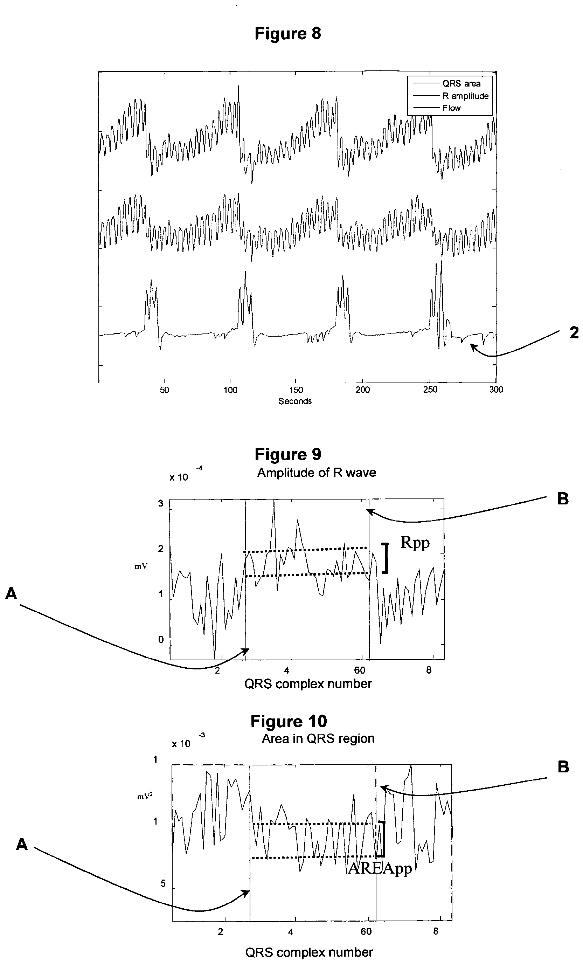

Sleep apnoea is often cyclic in nature and this can result in many of the extracted ECG signals also presenting a cyclic pattern as well which is shown particularly in the lowest trace of flow rate 2 of expired air in Figure 8. It can be observed that the QRS area and R wave amplitude also have a periodic pattern of the same frequency. (The parameters of an ECG signal are shown as the trace 1 in Figure 3.) As the breathing signal develops a longer term periodic pattern the accompanying extracted ECG signal also develops a periodic pattern with the same frequency, which may be used as surrogate estimated of the breathing signal in the method of the invention. Fourier analysis techniques and smoothing techniques using digital filtering may be used in the method to recognise these patterns. Envelopes that are found to contain these cyclic patterns are used to highlight regions of the data for closer examination as explained herein.

The method identifies the start and end points of respiratory events which have characteristics measurable in ECG signal data. Most conveniently, the ECG signal data are acquired by sensors adjacent a subject while the subject is asleep. The data is collected and stored in a computer file for manipulation with a microprocessor and computer programs to identify respiratory events, determine the respiratory effort, or calculate the apnoea/hypopnea index.

Table 1 provides definitions for some the parameters measurable in the ECG signal data and their acronyms used in this description. The parameters will be well known by persons skilled in the art of acquiring and analysing ECG signals. The method is not limited to the parameters in Table 1, but may include any ECG-derived signal which has a correlation with breathing effort.

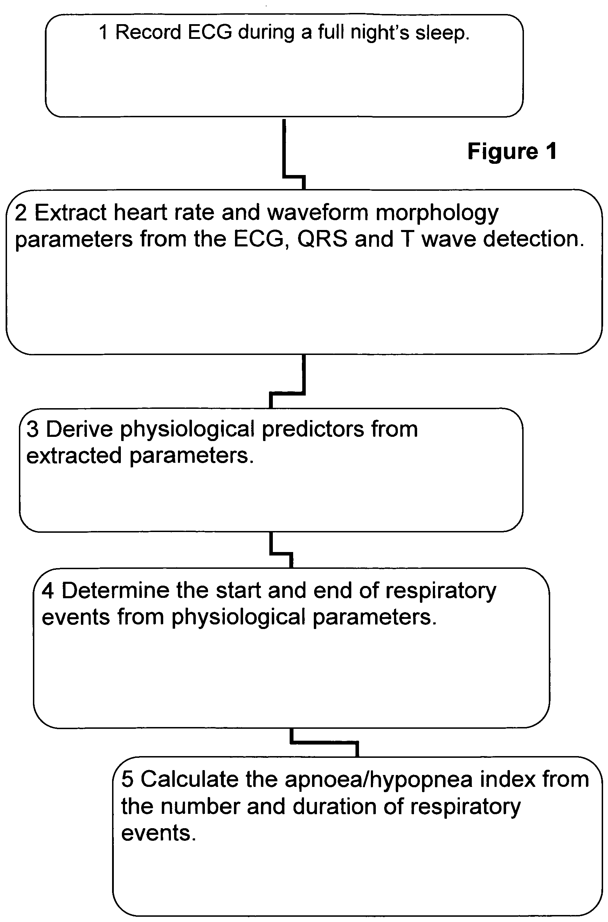

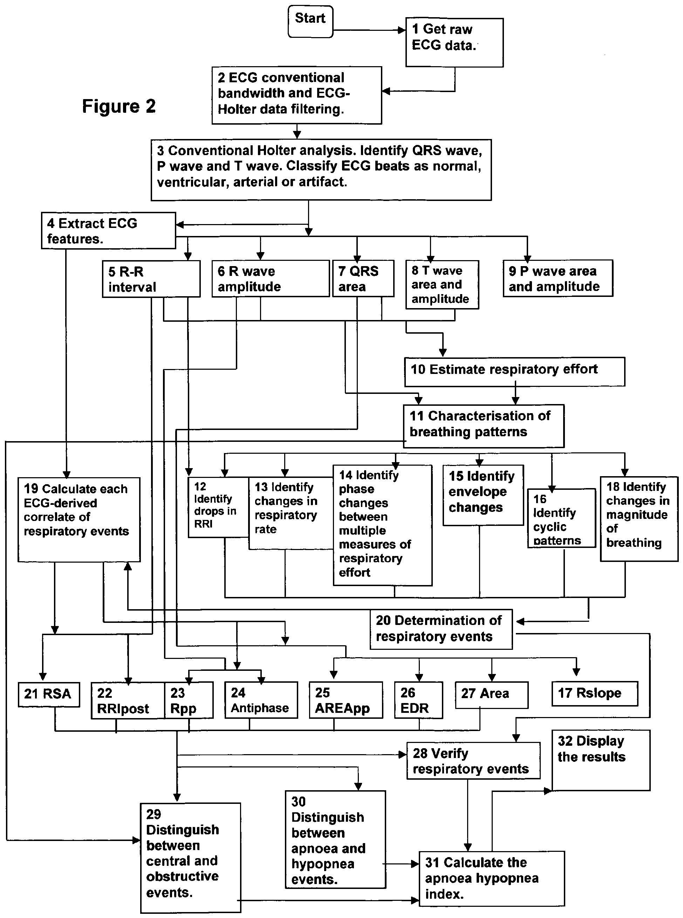

An overview of the steps of the method of the invention is presented in the flow-chart of Figure 1. A more detailed flow-chart further detailing the method is shown in Figure 2. A typical ECG trace 1 is shown in Figure 3 as a plot of acquired signal magnitude against time. All calculations of the method are carried out using a microprocessor. Referring to Figure 1, in a first step, the ECG signal is recorded from a subject using standard ECG monitoring equipment such as an ECG holter. Any suitable equipment that can record a digital ECG signal may be used. The acquired data is conveniently stored in a digital file on computer media for analysis. The data may be filtered according to a relevant bandwidth for ECG data using known techniques.

The ECG signal 1 over time has a characteristic wave-form morphology incorporating segments, shown in Figure 3, which are identified in the second step of the method. In particular, the peak commencing at point Q, reaching a maximum at R, and a minimum at S, is well known as the "QRS" complex. Further parameters used in the method and shown in the Figures are the amplitude and area under the graph of the T wave of each ECG complex, the amplitude of the signal at R, the time period between successive R waves, and the area under the graph of the QRS segment. The heart rate is measured as the time between R wave peaks shown in Figure 3 is known as the R-R interval (RRI).

The ECG-derived signals are used to generate a number ECG-derived correlates of respiratory events in a third step. These correlates are used to confirm changes that occur in the ECG during periods of respiratory or breathing events, including apnoea or hypopnea events. Each of the correlates that may be used in the method is described herein. The scope of the invention includes the use of any ECG-derived parameter that correlates with breathing events. The identified ECG parameters are then classified as being representative of normal or abnormal heart function. This analysis uses linear approximation to identify the various regions of the ECG complex. However, other relevant analyses may be used. The classification is carried out by examining changes to the ECG parameters identified in the second step.

The classification of a parameter as being normal or abnormal uses known correlations. For the classification of the heart rate, the method incorporates the following correlation. Respiratory Sinus Arrhythmia (RSA) is a naturally occurring rhythm observed in the heart rate (HR) or R-R interval (RRI) of the ECG, in mammals. This rhythm is the direct result of the interactions of the respiratory and the cardiovascular system. RSA is characterized by a periodic signal that displays maxima and minima in the RRI which is similar to the respiratory rate. Typically, the HR will increase with inspiration and decrease with expiration. Changes to the RSA signal may be used as a physiological predictor in the method.

It is well known that after a respiratory event there is a decrease in the RRI. This is associated with an increase in sympathetic activity during arousal. During an arousal there is a short period of increased heart rate, systolic blood pressure, and diastolic blood pressure accompanied by increased sympathetic activity. The heart rate variability is also reduced by the increase in sympathetic activity. This reduction in RRI may be used as a physiological predictor in the method.

The morphology of the ECG waveform is used to classify whether or not respiration is normal according to changes with respiration due to the motion of the heart with respect to the electrodes correlating with the changing intra-thoracic impedance. The rotation of the heart with respect to the electrodes, caused by breathing is evident in oscillations of both the amplitude of the R wave and the area of the R wave (this included the area of the whole QRS segment shown in Figure 3). As breathing is reduced or stops completely during an event a reduction in the peak to peak oscillations of the signal is expected. Changes to the amplitude and area may be used as physiological predictors in the method.

A sudden shift in orientation of the heart will cause a sudden change in the amplitude of the R wave. If the ECG leads are orthogonal the shift should be in the opposite directions. The opposite change in amplitude of the R wave in ECG leads may be used as a physical predictor in the method.

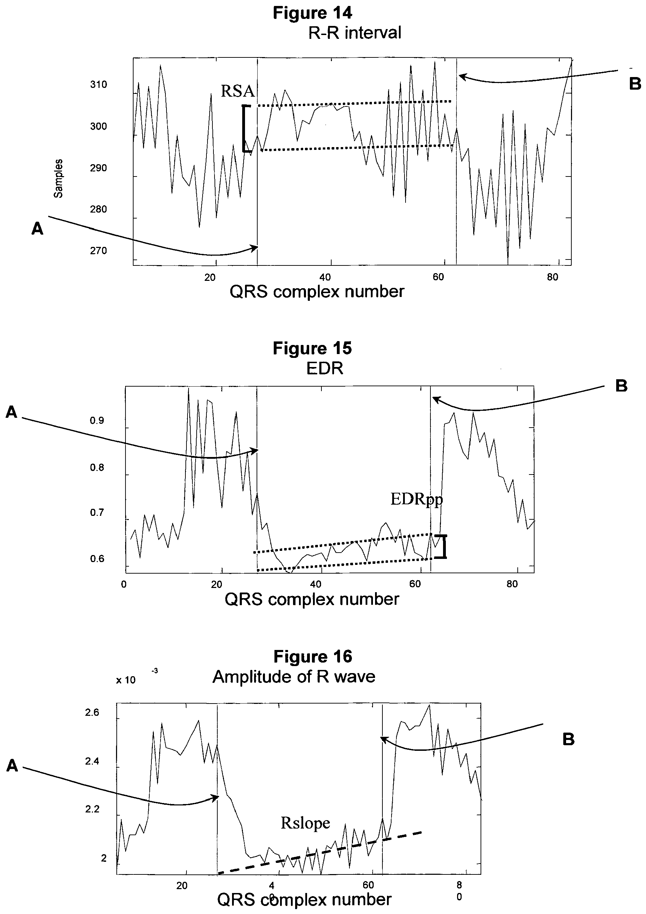

A gradual increase in the amplitude of the R or T wave may be used as a physical predictor. An example of an increasing R wave in an ECG signal is shown in Figure 16.

In a fourth step, by examining the changes in the physiological predictors for each ECG complex the method determines if each of the ECG complexes occurs at the start of a respiratory event. The start and end of each respiratory event is then determined in the fourth step of the method. Figure 2 shows the steps of the method for calculating the apnoea/hypopnea index from successive ECG complexes stored in a data file.

In a fifth step, the number of detected respiratory events and their duration is used calculate the apnoea/hypopnea index. The apnoea/hypopnea index is calculated as the average number of respiratory events that are longer than ten seconds, per hour.

The method advantageously uses ECG signal data alone to characterise breathing patterns. By examining the ECG derived respiratory effort and the extracted ECG signals, regions of data that contain changes in breathing patterns associated with respiratory events are identified. There are a number of changes described herein that may be investigated to help identify possible respiratory events.

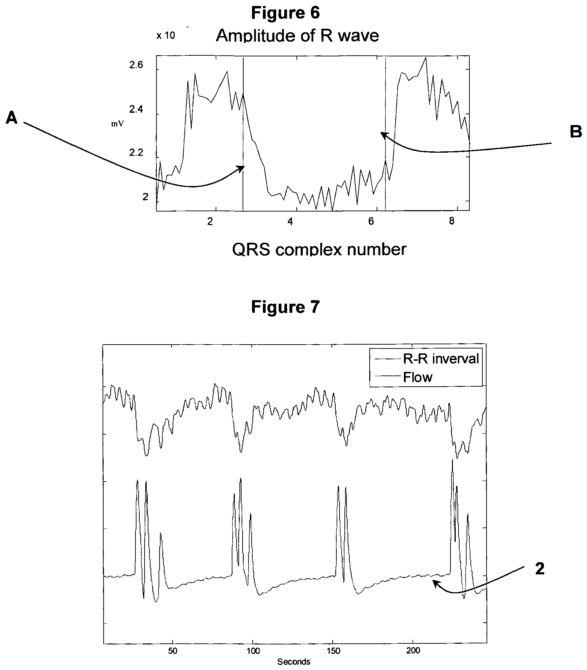

Figure 5 shows a graph of ECG signal parameters, QRS area and R-amplitude as well as the measured airflow 2 over an obstructive sleep apnoea event. There is expected to be a reduction in breathing magnitude during the event compared to before and after the event, which is clearly shown in Figure 5. Possible respiratory events are identified by examining the respiratory effort and locating regions that have a reduction in respiratory effort. The method uses identification of changes in the breathing rate to assist in marking possible respiratory events, i.e., the changes to the breathing rate that occur on event onset, during the event and on arousal. The method may use the sharp change in the in the QRS area and R wave amplitude occurs and the beginning and end of respiratory events. A sudden shift in orientation of the heart will cause a sudden change in the amplitude of the R wave. Sudden shift in the ECG-derived signals are used to mark the possible beginning and end of respiratory events. This is shown in Figure 6, where the sudden shift that occurs during a respiratory event is evident in this segment if the R wave amplitude signal. The lines at A and B mark the beginning and end of the respiratory event respectively. Figure 7 shows the dips in the RRI signal can be observed after each obstructive apnoea event, evident in the flow.

The method may include determination of breathing patterns from the ECG-derived measures of respiratory effort. This may include patterns of reduction in the magnitude of respiratory effort as shown in Figure 5 or patterns of abrupt variation in the respiratory rate as shown in Figure 7 or patterns of abrupt variation in the underlying level of respiratory effort markers shown in Figure 6. The method may use patterns of change in phase relationships between multiple measures of respiratory efforts, patterns of consistent variation in the magnitude of respiratory effort estimate(s) over the course of a sequence of breaths, or cyclical patterns in the envelope of respiratory effort estimate(s) as shown in Figure 8.

More specifically, with reference to Figure 2 raw ECG data is obtained from the subject via a two-lead (or greater) ECG recording. This must use at least two ECG leads that are orthogonal. This data is saved to a digital storage device. The calculation of the AHI commences with a microprocessor programmed to access and analyse the stored ECG signal data (Box 1 Figure 2) and filtering, if necessary, for the relevant bandwidth (Box 2 Figure 2). It will be understood that the large amount of signal data acquired and transformations required is carried out with a microprocessor with processing speed and power known in the art to be required, but also programmed to acquire, store, and manipulate signals and data according to the method described herein.

The analysis of the stored data commences with the examination using conventional Holter analysis programs and the QRS wave, P wave and T wave segments of the ECG are identified (Box 3 Figure 2). Each segment of the heart beat is then classified as normal, ventricular, arterial or artefact. An example of the collected data is graphed in Figure 4, which shows a representation of extracted ECG features from a sleeping subject breathing normally. The signal displayed against time in Figure 4 is that acquired from lead I of an ECG lead configuration known in the art. By examining properties of the identified waves ECG derived signals are generated by the software. The generated ECG derived signals are; R wave amplitude, QRS area, R-R interval (RRI), T wave amplitude, and T wave area. The first two signals, as well as the measured respiratory airflow are shown in Figure 4. The software program extracts morphological features of the ECG signal(s) (Box 4, Figure 2) including: start times, end times and peak times for the P-waves, QRS complexes and T-waves by means of morphological analysis of the ECG signal(s). This includes classifying QRS complexes as normal, ventricular, supraventricular or artifact by means of applying arrhythmia classification rules. Generating quantitative parameters of the ECG signal(s) including R-R intervals, R-wave amplitude, QRS complex area, T-wave amplitude and T-wave area (Boxes 5-9, Figure 2).

Many of the ECG derived signals demonstrate oscillatory patterns similar to the oscillatory patterns that occur with breathing (Figure 4). The breathing effort is estimated from the QRS area and R wave amplitude which oscillate with breathing (Boxes 10-11, Figure 2). The breathing signal, or ECG-Derived Respiration signal (EDR) may be calculated from the ratio of the areas of the QRS segment from orthogonal ECG leads (Box 26 Figure 2).

Local peaks that are associated with changes in breathing direction (changing from exhalation to inhalation or vice versa) are identified in each of the ECG derived signals (Box 10 Figure 2). These peaks are identified by examining oscillatory patterns, similarity of periods and signal magnitudes for respiratory peak candidates within there ECG derived signals.

Once these peaks have been identified in each of the ECG derived signals they are compared between signals. This enables a cross validation of the respiratory peaks in each signal (Box 10 Figure 2). By examining and comparing each of the peaks in each signal the single set of respiratory peaks are identified. This achieved by comparing the period between each of the peaks and the changes in amplitude of the peaks between signals (Box 10 Figure 2). By examining; the changes in the ECG derived signals and the estimated respiratory effort using the methods described above, markers for the beginning and end of possible ECG events are identified. These markers of possible beginning and end of events are used to generate a set of respiratory event candidates. The respiratory events are compared with the pre-defined or adjustable rules for the detected patterns of estimated breathing and R-R intervals including application of look-up tables and artificial neural networks.

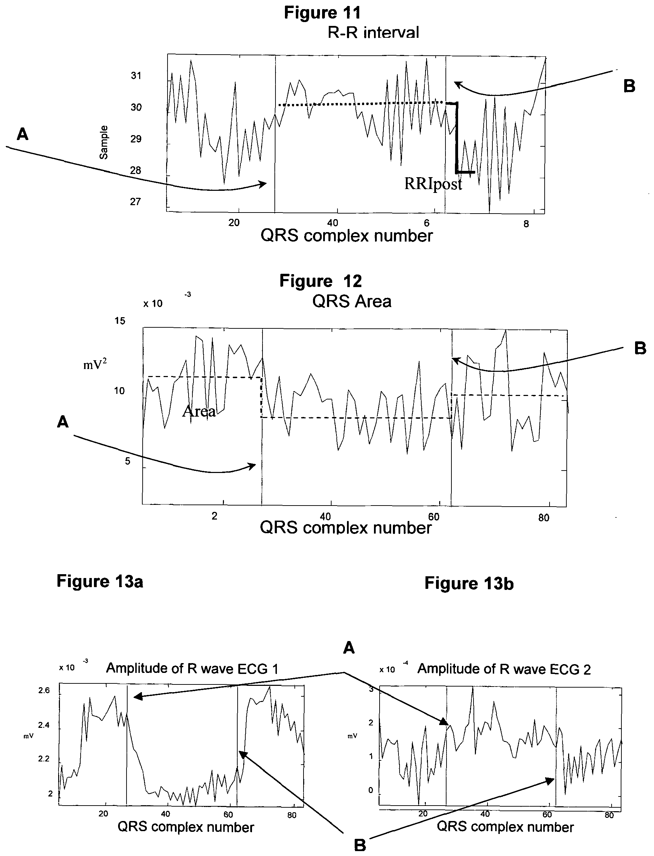

The method calculates the following parameters. The Reduction in the average peak to peak magnitude of oscillations of the R wave amplitude over the course of a potential respiratory event relative to the respective values before and after the event is shown in Figure 9. The reduction in the average peak to peak magnitude of oscillations of the area under the QRS complex over the course of a potential respiratory event relative to the respective values before and after the event is also shown in Figure 10. The reduction in the average R-R interval after a potential respiratory event relative to the average R-R interval over the course of the event is shown in Figure 11. The variation in the average value of the area under QRS complex over the course of potential respiratory event relative to respective values before and after the event is shown in Figure 12. The difference between variations in the average values of R wave amplitude over the course of potential respiratory event relative to the respective values before and after the event for pairs of multiple ECG signals as shown in Figure 13. The reduction in the average peak to peak magnitude of oscillations of R-R interval over the course of potential respiratory event relative to the respective values before and after the event as shown in Figure 14. The reduction in the average peak to peak magnitude of the ratio of areas under QRS complexes detected from the two orthogonal ECG leads over the course of potential respiratory event relative to the respective values before and after the event as shown in Figure 15. The estimate of linear trend for the R wave amplitude by means of fitting the least squares error linear approximation over the course of potential respiratory event is shown in Figure 16.

For each of the respiratory event candidates each of the ECG derived correlates of respiratory events are generated (Boxes 17, 19, 21-27 Figure 2). These correlates are used to either confirm or reject each of the respiratory event candidates. The respiratory event candidates that demonstrate that ECG derived correlates of respiratory events that generate the expected are accepted.

Once the respiratory peaks have been verified they can be used to generate an estimation of respiratory effort (Box 28 Figure 2). The respiratory effort is estimated by interpolating the sequences of values of R-wave amplitudes and QRS-complex areas into respective channels for from single or multiple ECG signals. These interpolated channels serve as approximate markers of respiratory effort. Sequences of respiratory peaks from individual approximate markers of respiratory effort are determined by means of detecting peaks on those markers with similar distances, signal variations, and slopes. Fine tuning sequences of respiratory peaks by means of matching the peak sequences determined from the individual markers to eliminate false positive respiratory peaks and recover missing respiratory peaks.

The rotation of the heart with respect to the electrodes, caused by breathing is evident in oscillations of both the amplitude of the R wave and the area of the R wave (this included the area of the whole QRS segment). As breathing is reduced or stops completely during an event a reduction in the peak to peak oscillations of the signal would be expected. As shown in Figures 9 and 10, the *Rpp* and the *AREApp* correlates are calculated by finding the value of the mean peak to peak oscillations of the R wave amplitude / QRS area during the event minus the value of the mean peak to peak oscillations of the R wave amplitude / QRS area before and after the event. Figure 9 shows the reduction in oscillations can be seen in the amplitude of the R wave during the respiratory event. The lines, A B, respectively, mark the beginning and end of the respiratory event. Figure 10 shows the reduction in oscillations can be seen in the area of the QRS segment during the respiratory event. The lines, A B, respectively, mark the beginning and end of the respiratory event.

It is well known that after a respiratory event there is a decrease in the RR-interval. This is associated with an increase in sympathetic activity during arousal. During an arousal there is a short period of increased heart rate, systolic blood pressure, and diastolic blood pressure accompanied by increased sympathetic activity. The heart rate variability is also reduced by the increase in sympathetic activity. The difference between the mean value of the RRI during the event and the mean value of the RRI after the event was used to calculate the *RRI_post* correlate which may be used in the method. Figure 11 shows the reduction in the R-R interval directly after respiratory event can be observed. The lines A, B, respectively, mark the beginning and end of the respiratory event.

A shift in orientation of the heart causes a change in the QRS area and R wave amplitude. The difference between the mean QRS area during the event and the mean QRS before and after the event is used to calculate the AREA correlate. Figure 12 shows the change in mean QRS area during the respiratory event. The lines, A, B, respectively mark the beginning and end of the respiratory event.

The difference between the mean R wave amplitude during the event and the mean R wave amplitude directly preceding and succeeding the event was calculated for two channels and then multiplied together to calculate the Antiphase correlate. If the ECG leads are orthogonal the shift should be in the opposite directions.

Figure 13 shows a sudden change in R wave amplitude being evident and the change in each lead is in opposite directions. The lines, A, B, respectively mark the beginning and end of the respiratory event

Respiratory Sinus Arrhythmia (RSA) is a naturally occurring rhythm observed in the heart rate (HR) or R-R interval (RRI) of the ECG. This rhythm is the direct result of the interactions of the respiratory and the cardiovascular systems. RSA is characterized by a periodic signal that displays maxima and minima in the RRI which is similar to the respiratory rate. Typically the HR will increase with inspiration and decrease with expiration. As demonstrated in, the difference of the value of the mean peak to peak oscillations of the RRI during the event and the value of the mean peak to peak oscillations of the RRI before and after the event was used to calculate the RSA correlate.

Figure 14 shows the reduction in oscillations of the R-R interval during the respiratory event can be observed. The lines A, B, respectively mark the beginning and end of the respiratory event.

The ECG Derived Respiration signal (EDR) is calculated as the ratio of the areas of the QRS segment from orthogonal ECG leads to determine a respiratory signal. The difference of the mean peak to peak oscillations of the EDR signal during the event and the value of the mean peak to peak oscillations of the EDR signal before and after the event is used to calculate the EDRpp correlate. Figure 15 shows the reduction in oscillations of the EDR signal during the respiratory event can be observed. The lines A, B, respectively, mark the beginning and end of the respiratory event.

Figure 16 shows an example ECG signal data where during the event there was a gradual increase in the amplitude of the R waves. The slope of the linear best fit, shown by the dashed line, for the R wave amplitude during the event may be used to calculate the *Rslope* correlate.

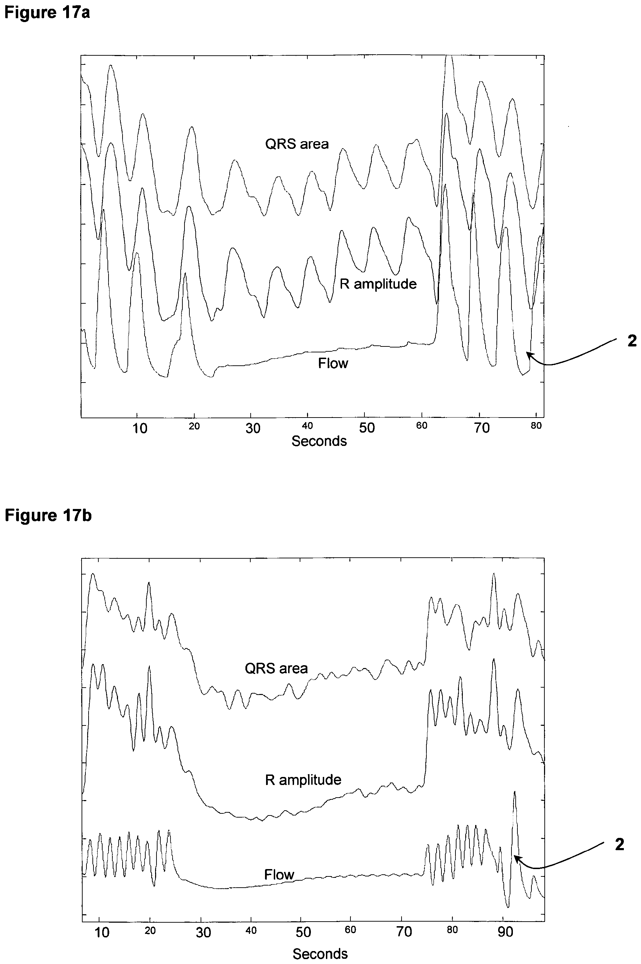

The respiratory effort from the QRS area and R wave amplitude enable event classification. As these signals can be used to estimate respiratory effort they can be used to assist in distinguishing between central events and obstructive events (Box 29 Figure 2). Respiratory effort should be evident and possibly increase during an obstructive event, while during a central event the respiratory effort should remain very low or nonexistent. Examples of this are presented in Figures 17a and b, which show differences between ECG parameters in OSA and CSA events. Figure 17a how the QRS area and R wave change during an obstructive event. It is evident that respiratory effort remains during the event. Figure 17b shows the QRS area and R wave change during a CSA event. It is evident that respiratory effort is dramatically reduced or non-existent. Also, ECG derived correlates of respiratory events yield different results depending on the type of event examined. For example the AREApp correlate would expect to have a large magnitude for a central event than an obstructive event as there should be a greater reduction in the oscillations of the QRS area that are correlated with breathing.

As there is a greater reduction in breathing during apnoea events compared to hypopnea events, larger changes in ECG derived correlates of respiratory events are expected in apnoea events than with hypopnea events. By quantifying these changes in ECG derived correlates of respiratory events the method may discriminate between apnoea and hypopnea events. The method may discriminate between apnoea and hypopnea events by calculating the ratios of average oscillation magnitudes within the respiratory events to the respective values before and after respiratory events for the ECG derived measures of respiratory effort with the events that have these ratios below the predetermined thresholds being classified as apnoeas and the other events classified as hypopneas.

The AHI is calculated dividing the total number of respiratory events by the number of hours of ECG signal recording period (Box 31 Figure 2). Calculating the apnoea/hypopnea index for the sleep evaluation time interval provides a clinical estimate of the presence, severity and type of sleep apnoea by means Separate indices may being calculated for obstructive, central and combination of obstructive and mixed respiratory events. Preferably, the index is displayed on a suitable monitor for viewing by a user of the method.

**TABLE 1: SUMMARY OF THE DESCRIPTION OF ECG DERIVED CORRELATES OF RESPIRATORY EVENTS**

| **Description of ECG derived correlates of respiratory events** | **Physiological Predictor** |
|---|---|
| Change in peak to peak oscillations of the EDR signal during the event. | **EDR** |
| Measure of the sudden opposite change of R wave amplitude between 2 orthoganal ECG leads during the event. | **Antiphase** |
| Change in RSA signal (peak to peak oscillations of the RRI signal). | **RSA** |
| Change in the mean RRI signal after the event. | **RRIpost** |
| Change in peak to peak oscillations of the R amplitude during the event. | **Rpp** |
| Change in peak to peak oscillations of the QRS area during the event. | **AREApp** |
| The change in the QRS area during the event. | **Area** |
| Measure of the increase in amplitude of the R wave during the event. | **Rslope** |

### References

[1] "Sleep-related breathing disorders in adults: recommendations for syndrome definition and measurement techniques in clinical research. The Report of an American Academy of Sleep Medicine Task Force," Sleep, vol. 22, pp. 667-89, Aug 1 1999.
[2] M. Schrader, C. Zywietz, V. von Einem, B. Widiger, and G. Joseph, "Detection of sleep apnea in single channel ECGs from the PhysioNet data base," 2000, pp. 263-266.
[3] C. Maier, H. Dickhaus, M. Bauch, and T. Penzel, "Comparison of heart rhythm and morphological ECG features in recognition of sleep apnea from the ECG," in Computers in Cardiology, 2003, 2003, pp. 311-314.
[4] S. Vijendra, K. Behbehani, E. A. Lucas, J. R. Burk, D. N. Burli, and D. H. Dao, "The use of R-wave morphology in the detection of sleep-disordered breathing using the electrocardiogram - a comparison between leads," 2004, pp. 3881-3884 Vol.6.
[5] F. Roche, V. Pichot, E. Sforza, I. Court-Fortune, D. Duverney, F. Costes, M. Garet, and J. C. Barthelemy, "Predicting sleep apnoea syndrome from heart period: a time-frequency wavelet analysis," Eur Respir J, vol. 22, pp. 937-42, Dec 2003.
[6] J. D. Lattimore, D. S. Celermajer, and I. Wilcox, "Obstructive sleep apnea and cardiovascular disease," J Am Coll Cardiol, vol. 41, pp. 1429-37, May 7 2003.
[7] J. Wolf, J. Lewicka, and K. Narkiewicz, "Obstructive sleep apnea: An update on mechanisms and cardiovascular consequences," Nutr Metab Cardiovasc Dis, Feb 19 2007.
[8] M. T. Naughton, "Heart failure and obstructive apnoea," Sleep Medicine Reviews, vol. 2, pp. 93-103, 1998.
[9] P. Grossman and E. W. Taylor, "Toward understanding respiratory sinus arrhythmia: relations to cardiac vagal tone, evolution and biobehavioral functions," Biol Psychol, vol. 74, pp. 263-85, Feb 2007.
[10] J. W. Denver, S. F. Reed, and S. W. Porges, "Methodological issues in the quantification of respiratory sinus arrhythmia," Biol Psychol, vol. 74, pp. 286-94, Feb 2007.
[11] F. Yasuma and J. Hayano, "Respiratory sinus arrhythmia: why does the heartbeat synchronize with respiratory rhythm?," Chest, vol. 125, pp. 683-90, Feb 2004.
[12] H. J. Burgess, J. Kleiman, and J. Trinder, "Cardiac activity during sleep onset," Psychophysiology, vol. 36, pp. 298-306, May 1999.
[13] M. J. Carrington, R. Barbieri, I. M. Colrain, K. E. Crowley, Y. Kim, and J. Trinder, "Changes in cardiovascular function during the sleep onset period in young adults," J Appl Physiol, vol. 98, pp. 468-76, Feb 2005.
[14] R. J. Davies, P. J. Belt, S. J. Roberts, N. J. Ali, and J. R. Stradling, "Arterial blood pressure responses to graded transient arousal from sleep in normal humans," J ApplPhysiol, vol. 74, pp. 1123-30, Mar 1993.
[15] K. Narkiewicz, N. Montano, C. Cogliati, P. J. van de Borne, M. E. Dyken, and V. K. Somers, "Altered cardiovascular variability in obstructive sleep apnea," Circulation, vol. 98, pp. 1071-7, Sep 15 1998.
[16] G. B. Moody, R. G. Mark, A. Zoccola, and S. Mantero, "Derivation of Respiratory Signals from Multi-lead ECGs," Computers in Cardiology, vol. 12, pp. 113-116, 1985.

## Claims

1. Apparatus for determining respiratory effort from an ECG signal comprising: at least two ECG leads for acquiring at least two orthogonal signals from a subject; means for transforming said signals to digital data; electronic data storage means; and a microprocessor programmed to extract waveform morphology data from said digital data and derive physiological predictors from the extracted waveform morphology data, **characterized in that** the microprocessor is further programmed to determine the start and end of respiratory events from either or both of a sharp change in the QRS area and a sharp change in the R-wave amplitude, generate a set of respiratory event candidates, and generate ECG derived correlates for each respiratory event candidate, wherein the correlates are any one or more of a sudden and opposite change in amplitude of an R-wave between two of the orthogonal ECG leads, a change in peak-to-peak oscillations of the RRI signal, and a change in the mean RRI signal, wherein a sudden and opposite change in amplitude of an R-wave between two of the orthogonal ECG leads is derived by calculating the difference of a value of the mean peak to peak oscillations of the R wave amplitude, a change in peak-to-peak oscillations of the RRI signal is derived by calculating a change in peak-to-peak oscillations of the QRS area during the event, and a change in the mean RRI signal is derived by calculating a change in a value of the mean peak to peak oscillations of the R wave amplitude before and after the event, and comparing the correlates of the respiratory event candidates with pre-defined rules for the detected patterns of estimated breathing and RRI to confirm or reject each of the set of respiratory event candidates to determine respiratory events and respiratory effort.

2. Apparatus according to claim 1, wherein the microprocessor is further programmed to cross validate peaks of the respiratory effort between signals to generate the set of respiratory event candidates.

3. Apparatus according to any preceding claim, wherein said derived physiological predictors include ECG-derived respiration signal and heart rate.

4. Apparatus according to any preceding claim, further comprising a microprocessor programmed to calculate an apnoea/hypopnea index.

5. A method for determining respiratory events from an ECG signal, comprising the steps of: providing an apparatus according to any one of claims 1 to 4; acquiring said ECG signal data; operating said microprocessor to extract said waveform morphology data from said ECG signal data, and derive physiological predictors from the extracted waveform morphology data, **characterized by** the step of operating said microprocessor to determine the start and end of respiratory events from either or both of a sharp change in the QRS area and a sharp change in the R-wave amplitude, generate a set of respiratory event candidates, and generate ECG derived correlates for each respiratory event candidate, wherein the correlates are any one or more of a sudden and opposite change in amplitude of an R-wave between two of the orthogonal ECG leads, a change in peak-to-peak oscillations of the RRI signal, and a change in the mean RRI signal, wherein a sudden and opposite change in amplitude of an R-wave between two of the orthogonal ECG leads is derived by calculating the difference of a value of the mean peak to peak oscillations of the R wave amplitude, a change in peak-to-peak oscillations of the RRI signal is derived by calculating a change in peak-to-peak oscillations of the QRS area during the event, and a change in the mean RRI signal is derived by calculating a change in a value of the mean peak to peak oscillations of the R wave amplitude before and after the event, comparing the correlates of the respiratory event candidates with pre-defined rules for the detected patterns of estimated breathing and RRI to confirm or reject each of the set of respiratory event candidates, and estimate the respiratory events and respiratory effort.

6. The method of claim 5, further comprising the step of operating the microprocessor to cross validate peaks of the respiratory effort between signals to generate the set of respiratory event candidates.

7. The method of either one of claims 5 and 6, further comprising the step of operating the microprocessor to classify a respiratory event as one of apnoea, in which there is a large change in said waveform morphology data, or hypopnea, in which there is a small change in said waveform morphology data.

8. The method of any one of claims 5 to 7, further comprising the step of operating the microprocessor to classify a respiratory event as one of obstructive apnoea, in which respiratory effort increases, or central apnoea, in which respiratory effort remains very low or non-existent.

9. The method of any one of claims 5 to 8, further comprising the step of operating the microprocessor to calculate an apnoea/hypopnea index.

10. The method of any one of claims 5 to 9, wherein said microprocessor is operated to transform said waveform data into any one, or combination, of ECG-derived physiological predictors including ECG-derived respiration signal and heart rate.

## Patentansprüche

1. Vorrichtung zur Bestimmung der Atemleistung aus einem ECG-Signal umfassend:
wenigstens zwei ECG-Spitzem, um wenigstens zwei orthogonale Signale von einer Versuchsperson zu erhalten;
Mittel zur Umwandlung dieser Signale in digitale Daten;
Mittel zum Speichern elektronischer Daten
und einen Mikroprozessor, der programmiert ist, um Morphologiedaten einer Wellenform aus diesen digitalen Daten zu extrahieren und physiologische Vorhersagen aus den extrahierten Morphologiedaten der Wellenform abzuleiten,
**gekennzeichnet dadurch, dass** der Mikroprozessor weiterhin programmiert ist, um den Beginn und das Ende von Atemereignissen aus einer oder beiden Möglichkeiten umfassend eine scharfe Änderung im QRS-Bereich und eine scharfe Änderung in der R-Wellen-Amplitude zu bestimmen,
einen Satz von Anwärtern für Atemwegsereignisse zu erzeugen
und vom ECG abgeleitete Korrelate für jeden Anwärter eines Atemwegsereignisses zu erzeugen,
wobei die Korrelate eines oder mehrere sind ausgewählt aus
einer plötzlichen und entgegengesetzten Änderung der Amplitude einer R-Welle zwischen zwei der orthogonalen ECG-Spitzen,
einer Änderung in einer Oszillation eines RRI-Signals von Peak zu Peak
und einer Änderung in dem mittleren RRI-Signal,
wobei eine plötzliche und entgegengesetzte Änderung in der Amplitude einer R-Welle zwischen zwei der orthogonalen ECG-Spitzen abgeleitet wird durch Berechnen der Differenz zwischen einem Wert der mittleren Oszillationen von Peak zu Peak der R-Wellen-Amplitude,
eine Änderung in den Peak zu Peak Oszillationen des RRI-Signals abgeleitet wird durch Berechnen einer Änderung in den Oszillationen von Peak zu Peak eines QRS-Bereichs während des Ereignisses und eine Änderung in dem mittleren RRI-Signal abgeleitet wird durch Berechnen einer Änderung in einem Wert der mittleren Peak zu Peak Oszillationen der R-Wellen-Amplitude vor und nach dem Ereignis und vergleichen der Korrelate der Anwärter für Atemwegsereignisse mit vorgegebenen Regeln für festgestellte Muster angenommener Atmung und RRI, um jeden Satz von Anwärtern für Atemwegsereignisse zu bestätigen oder auszuschließen, um Atemwegsereignisse und Atemleistung zu bestimmen.

2. Vorrichtung nach Anspruch 1, bei der der Mikroprozessor weiterhin programmiert ist, um Peaks der Atemleistung zwischen Signalen kreuzweise zu validieren, um einen Satz von Anwärtern von Atemwegsereignissen zu erzeugen.

3. Vorrichtung nach einem der vorhergehenden Ansprüche, bei der diese abgeleiteten physiologischen Vorhersagen vom ECG abgeleitetes Atemsignal und Herzfrequenz umfassen.

4. Vorrichtung nach einem der vorhergehenden Ansprüche, welche weiterhin einen Mikroprozessor umfasst, welcher programmiert ist, einen Apnoe/Hypopnoe-Index zu berechnen.

5. Verfahren zur Bestimmung von Atemwegsereignissen aus einem ECG-Signal, umfassend die Schritte:
bereitstellen einer Vorrichtung gemäß einem der Ansprüche 1 bis 4;
gewinnen der ECG Signaldaten;
betreiben des Mikroprozessors, um die Morphologiedaten für die Wellenform aus den ECG Signaldaten zu extrahieren
und ableiten physiologischer Vorhersagen aus den extrahierten Morphologiedaten der Wellenform,
**gekennzeichnet durch** den Schritt des Betreibens des Mikroprozessors zur Bestimmung des Beginns und des Endes von Atemwegsereignissen ausgewählt aus einem oder beiden, einer scharfen Änderung im QRS Bereich und einer scharfen Änderung in der R-Wellen-Amplitude,
erzeugen eines Satzes von Anwärtern für Atemwegsereignisse
und erzeugen vom ECG abgeleiteter Korrelate für jeden Anwärter eines Atemwegsereignisses,
wobei die Korrelate eines oder mehrere sind ausgewählt aus
einer plötzlichen und entgegengesetzten Änderung der Amplitude einer R-Welle zwischen zwei der orthogonalen ECG-Spitzen,
einer Änderung in einer Oszillation eines RRI-Signals von Peak zu Peak
und einer Änderung in dem mittleren RRI-Signal,
wobei eine plötzliche und entgegengesetzte Änderung in der Amplitude einer R-Welle zwischen zwei der orthogonalen ECG-Spitzen abgeleitet wird durch berechnen der Differenz zwischen einem Wert der mittleren Oszillationen von Peak zu Peak der R-Wellen-Amplitude,
eine Änderung in den Peak zu Peak Oszillationen des RRI-Signals abgeleitet wird durch berechnen einer Änderung in den Oszillationen von Peak zu Peak eines QRS-Bereichs während des Ereignisses und eine Änderung in dem mittleren RRI-Signal abgeleitet wird durch berechnen einer Änderung in einem Wert der mittleren Peak zu Peak Oszillationen der R-Wellen Amplitude vor und nach dem Ereignis und vergleichen der Korrelate der Anwärter für Atemwegsereignisse mit vorgegebenen Regeln für festgestellte Muster angenommener Atmung und RRI, um jeden Satz von Anwärtern für Atemwegsereignisse zu bestätigen oder auszuschließen, um Atemwegsereignisse und Atemleistung zu bestimmen.

6. Verfahren nach Anspruch 5, weiterhin umfassend den Schritt des Betreibens des Mikroprozessors um Peaks der Atemleistung zwischen Signalen kreuzweise zu validieren, um einen Satz von Anwärtern von Atemwegsereignissen zu erzeugen.

7. Verfahren nach einem der Ansprüche 5 oder 6, weiterhin umfassend den Schritt des Steuerns des Mikroprozessors, um ein Atemwegsereignis als Apnoe zu klassifizieren, bei dem es eine große Veränderung in den Morphologiedaten der Wellenform gibt oder als Hypopnoe, bei dem es eine geringe Änderung in den Morphologiedaten der Wellenform gibt.

8. Verfahren nach einem der Ansprüche 5 bis 7, weiterhin umfassend den Schritt des Steuerns des Mikroprozessors, um ein Atemwegsereignis als obstruktive Apnoe zu klassifizieren, bei dem die Atemleistung ansteigt, oder zentrale Apnoe, bei dem die Atemleistung sehr niedrig ist oder nicht-existent ist.

9. Verfahren nach einem der Ansprüche 5 bis 8, weiterhin umfassend den Schritt des Betreibens des Mikroprozessors, um einen Apnoe/Hypopnoe Index zu berechnen.

10. Verfahren nach einem der Ansprüche 5 bis 9, bei dem der Mikroprozessor gesteuert wird, um die Wellenformdaten in irgendeine oder eine Kombination von ECG-abgeleiteten physiologischen Vorhersagen umzuwandeln, einschließlich ECG-abgeleiteter Atemwegssignale und Herzfrequenzen.

## Revendications

1. Appareil pour déterminer un effort respiratoire à partir d'un signal ECG comprenant : au moins deux électrodes d'ECG pour acquérir au moins deux signaux orthogonaux à partir d'un sujet ; un moyen pour transformer lesdits signaux en données numériques ; un moyen de stockage de données électronique ; et un microprocesseur programmé pour extraire des données de morphologie de forme d'onde à partir desdites données numériques et déduire des indicateurs physiologiques à partir des données de morphologie de forme d'onde extraites, **caractérisé par le fait que** le microprocesseur est en outre programmé pour déterminer le début et la fin d'événements respiratoires à partir de l'un ou l'autre, ou des deux, d'un changement brusque de l'aire QRS et d'un changement brusque de l'amplitude d'onde R, générer un ensemble de candidats d'événement respiratoire, et générer des corrélations déduites d'ECG pour chaque candidat d'événement respiratoire, les corrélations étant un ou plusieurs parmi un changement soudain et opposé d'amplitude d'une onde R entre deux des électrodes d'ECG orthogonales, un changement d'oscillations pic à pic du signal RRI, et un changement du signal RRI moyen, un changement soudain et opposé d'amplitude d'une onde R entre deux des électrodes d'ECG orthogonales étant déduit par calcul de la différence d'une valeur des oscillations pic à pic moyennes de l'amplitude d'onde R, un changement d'oscillations pic à pic du signal RRI étant déduit par calcul d'un changement d'oscillations pic à pic de l'aire QRS pendant l'événement, et un changement du signal RRI moyen étant déduit par calcul d'un changement d'une valeur des oscillations pic à pic moyennes de l'amplitude d'onde R avant et après l'événement, et comparer les corrélations des candidats d'événement respiratoire à des règles prédéfinies pour les motifs détectés de respiration et RRI estimés pour confirmer ou rejeter chacun de l'ensemble de candidats d'événement respiratoire afin de déterminer des événements respiratoires et un effort respiratoire.

2. Appareil selon la revendication 1, dans lequel le microprocesseur est en outre programmé pour réaliser une validation croisée des pics de l'effort respiratoire entre des signaux pour générer l'ensemble de candidats d'événement respiratoire.

3. Appareil selon l'une quelconque des revendications précédentes, dans lequel lesdits indicateurs physiologiques déduits comprennent un signal de respiration et une fréquence cardiaque déduits d'ECG.

4. Appareil selon l'une quelconque des revendications précédentes, comprenant en outre un microprocesseur programmé pour calculer un indice d'apnées/hypopnées.

5. Procédé pour déterminer des événements respiratoires à partir d'un signal ECG, comprenant les étapes : disposer un appareil selon l'une quelconque des revendications 1 à 4 ; acquérir lesdites données de signal ECG ; actionner ledit microprocesseur pour extraire des données de morphologie de forme d'onde à partir desdites données numériques, et déduire des indicateurs physiologiques à partir des données de morphologie de forme d'onde extraites, **caractérisé par** le fait l'étape d'actionnement dudit microprocesseur pour déterminer le début et la fin d'événements respiratoires à partir de l'un ou l'autre, ou des deux, d'un changement brusque de l'aire QRS et d'un changement brusque de l'amplitude d'onde R, générer un ensemble de candidats d'événement respiratoire, et générer des corrélations déduites d'ECG pour chaque candidat d'événement respiratoire, les corrélations étant un ou plusieurs parmi un changement soudain et opposé d'amplitude d'une onde R entre deux des électrodes d'ECG orthogonales, un changement d'oscillations pic à pic du signal RRI, et un changement du signal RRI moyen, un changement soudain et opposé d'amplitude d'une onde R entre deux des électrodes d'ECG orthogonales étant déduit par calcul de la différence d'une valeur des oscillations pic à pic moyennes de l'amplitude d'onde R, un changement d'oscillations pic à pic du signal RRI étant déduit par calcul d'un changement d'oscillations pic à pic de l'aire QRS pendant l'événement, et un changement du signal RRI moyen étant déduit par calcul d'un changement d'une valeur des oscillations pic à pic moyennes de l'amplitude d'onde R avant et après l'événement, comparer les corrélations des candidats d'événement respiratoire à des règles prédéfinies pour les motifs détectés de respiration et RRI estimés pour confirmer ou rejeter chacun de l'ensemble de candidats d'événement respiratoire, et estimer les événements respiratoires et l'effort respiratoire.

6. Procédé selon la revendication 5, comprenant en outre l'étape d'actionnement du microprocesseur pour réaliser une validation croisée de pics de l'effort respiratoire entre des signaux pour générer l'ensemble de candidats d'événement respiratoire.

7. Procédé selon l'une des revendications 5 et 6, comprenant en outre l'étape d'actionnement du microprocesseur pour classer un événement respiratoire en tant que l'une parmi une apnée, dans laquelle il y a un changement important desdites données de morphologie de forme d'onde, ou une hypopnée, dans laquelle il y a un changement faible desdites données de morphologie de forme d'onde.

8. Procédé selon l'une quelconque des revendications 5 à 7, comprenant en outre l'étape d'actionnement du microprocesseur pour classer un événement respiratoire en tant que l'une parmi une apnée obstructive, dans laquelle un effort respiratoire augmente, ou une apnée centrale, dans laquelle un effort respiratoire reste très faible ou est non-existant.

9. Procédé selon l'une quelconque des revendications 5 à 8, comprenant en outre l'étape d'actionnement du microprocesseur pour calculer un indice d'apnées/hypopnées.

10. Procédé selon l'une quelconque des revendications 5 à 9, dans lequel ledit microprocesseur est actionné pour transformer lesdites données de forme d'onde en l'un quelconque, ou une combinaison, d'indicateurs physiologiques déduits d'ECG, y compris un signal de respiration et une fréquence cardiaque déduits d'ECG.
